(19) Europäisches Patentamt
European Patent Office
Office européen des brevets

(11) Veröffentlichungsnummer: **0 455 076 A2**

(12) **EUROPÄISCHE PATENTANMELDUNG**

(21) Anmeldenummer: **91106378.2**

(51) Int. Cl.5: **C07D 331/02**, C08C 19/30

(22) Anmeldetag: **20.04.91**

(30) Priorität: **03.05.90 DE 4014163**

(43) Veröffentlichungstag der Anmeldung:
**06.11.91 Patentblatt 91/45**

(84) Benannte Vertragsstaaten:
**DE FR GB IT**

(71) Anmelder: **BAYER AG**
**W-5090 Leverkusen 1 Bayerwerk(DE)**

(72) Erfinder: **Böhm, Stefan, Dr.**
**Carl-Leverkus-Strasse 30**
**W-5090 Leverkusen 1(DE)**
Erfinder: **Marhold, Albrecht, Dr.**
**Carl-Duisberg-Strasse 329**
**W-5090 Leverkusen 1(DE)**
Erfinder: **Greve, Heinz-Hermann, Dr.**
**Heidemannstrasse 76**
**W-5000 Köln 30(DE)**

(54) **1-Fluor-2,3-epithiopropan und daraus erhältliche Elastomere.**

(57) Die neue Verbindung 1-Fluor-2,3-epithiopropan und daraus erhältliche Elastomere.

EP 0 455 076 A2

Die vorliegende Erfindung betrifft die neue Verbindung 1-Fluor-2,3-epithiopropan, ein Verfahren zu deren Herstellung und daraus erhältliche Elastomere

Es ist bekannt, daß man mit geeigneten Schwefelverbindungen in Oxiranen das Sauerstoffatom gegen ein Schwefelatom austauschen kann (siehe Russ. Chem. Rev. 44 (2), 142 bis 144 (1975)). Für die Überführung von Epichlorhydrin in das entsprechende Ethylensulfid ist bekannt, daß die Gegenwart von Wasser während der Reaktion die Ausbeute vermindert und man mit Kaliumthiocyanat als Schwefel liefernder Verbindung in Abwesenheit von Wasser 1-Chlor-2,3-epithiopropan in einer Ausbeute von 47 % erhalten kann (siehe J. Chem. Soc. 1946, 1050 bis 1052).

Elastomere, die ihre Eigenschaften auch bei tiefen Temperaturen behalten, sind meist Polythioether, die halogenfrei sind. Nachteilig bei Polythioethern ist, daß diese im allgemeinen nicht im erwüschten Ausmaß ölbeständig sind.

Es wurde nun die neue Verbindung 1-Fluor-2,3-epithiopropan gefunden, die der Formel

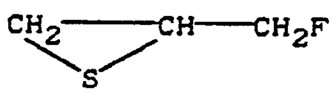

entspricht.

Der Siedepunkt und weitere charakteristische Parameter dieser Verbindung sind in den Beispielen angegeben.

Es wurde weiterhin ein Verfahren zur Herstellung von 1-fluor-2,3-epithiopropan gefunden, das dadurch gekennzeichnet ist, daß man 1-Fluor-2,3-epoxypropan mit einem Schwefel übertragenden Agens in Gegenwart von Wasser und einem polaren organischen Lösungsmittel umsetzt.

Das als Ausgangsprodukt benötigte 1-Fluor-2,3-epoxypropan ist bekannt und gut zugänglich (siehe z.B. J. Org. Chem. 45, 3930 (1980)).

Als Schwefel übertragende Agentien kommen beispielsweise Kaliumthiocyanat, Ammoniumthiocyanat, Thiocyansäure, Thioharnstoff, Arylthioharnstoffe, Diarylthioharnstoffe, Thiocarbonsäuren und Salze von Thiolsäuren (z.B. Kaliumsalze von Phosphorthiol- und/oder Phosphordithiolsäuren) in Betracht. Bevorzugt ist Kaliumthiocyanat.

Es ist im allgemeinen vorteilhaft, das Schwefel übertragende Agens in der stöchiometrisch erforderlichen Menge oder in mäßigem Überschuß einzusetzen, beispielsweise 1,0 bis 2,5 Äquivalente Schwefel übertragendes Agens pro Mol 1-Fluor-2,3-epoxypropan. Bevorzugt sind 1,0 bis 1,5 Äquivalente Schwefel übertragendes Agens pro Mol 1-Fluor-2,3-epoxypropan.

Das erfindungsgemäße Verfahren wird in Gegenwart von Wasser und einem polaren organischen Lösungsmittel durchgeführt. Als polare organische Lösungsmittel kommen beispielsweise Alkohole in Frage, insbesondere solche mit 1 bis 6 C-Atomen. Bevorzugt sind Methanol und Ethanol. Die relativen Volumenmengen von Wasser zu polarem organischen Lösungsmittel können beispielsweise zwischen 1:10 und 10:1 variiert werden, vorzugsweise zwischen 1:3 und 3:1.

Wasser wird vorzugsweise mindestens soviel zugesetzt, daß das Gemisch aus Wasser und polarem organischem Lösungsmittel zusammen mit dem eingesetzten Schwefel übertragenden Agens eine klare Lösung ergibt. Beispielsweise kann man auf 100 g Schwefel übertragendes Agens 50 bis 500 ml Wasser und 50 bis 500 ml polares organisches Lösungsmittel einsetzen.

Geeignete Temperaturen für die erfindungsgemäße Umsetzung sind beispielsweise solche von 0 bis 50 °C. Vorzugsweise wird bei Raumtemperatur gearbeitet, also beispielsweise bei 18 bis 25 °C.

Man kann das erfindungsgemäße Verfahren beispielsweise so durchführen, daß man zunächst Wasser, polares organisches Losungsmittel und Schwefel übertragendes Agens mischt und dieses Gemisch vorlegt, dann langsam und unter intensivem Rühren 1-Fluor-2,3-epoxypropan zufügt, nachdem dieses abreagiert hat (z.B. gaschromatographisch kontrolliert) feste Bestandteile abtrennt, diese mit einem wenig oder unpolaren Lösungsmittel wäscht, aus dem mit den Waschflüssigkeiten vereinigten Filtrat die organische Phase abtrennt, diese mit Wasser wäscht, trocknet und destilliert. Geeignete wenig oder unpolare Lösungsmittel sind beispielsweise gesättigte Kohlenwasserstoffe.

Es ist überraschend, daß man erfindungsgemäß trotz der Gegenwart von Wasser während der Reaktion 1-Fluor-2,3-epoxythiopropan in Ausbeuten von über 50 % der Theorie erhalten kann, denn gemäß dem eingangs geschilderten Stand der Technik wird in wasserfreiem Medium aus Epichlorhydrin und Kaliumthiocyanat das 1-Chlor-2,3-epithiopropan in 47 %iger Ausbeute, bei Gegenwart von Wasser in noch geringerer Ausbeute erhalten.

Es wurde weiterhin gefunden, daß man Elastomere erhält, wenn man 1-Fluor-2,3-epithiopropan stehen läßt. Dieses Stehenlassen kann beispielsweise zwischen 10 und 100 Stunden dauern bei Temperaturen von beispielsweise 10 bis 50 °C. So hergestellte Elastomere, die man nach dem Stehenlassen z.B. durch Abtrennen noch flüssiger Anteile und Trocknen des verbleibenden Feststoffs isolieren kann, zeichnen sich durch gute mechanische Eigenschaften, auch bei tiefen Temperaturen aus, was die ermittelte niedrige Glastemperatur belegt. Im Vergleich zu Polythioethern sind die aus 1-Fluor-2,3-epithiopropan erhältlichen Elastomere wesentlich besser ölbeständig.

Beispiele

Beispiel 1

152,2 g (2 Mol) 1-Fluor-2,3-epoxypropan wurden langsam bei Raumtemperatur zu einer Mischung aus 245 g (2,5 Mol) Kaliumthiocyanat, 200 ml Wasser und 150 ml Ethanol getropft, wobei stark gerührt wurde. Es wurde nachgerührt, bis im Reaktionsgemisch kein 1-Fluor-2,3-epoxypropan mehr gaschromatographisch nachgewiesen werden konnte, Dann wurde von dem gebildeten Niederschlag abdekantiert, mit Pentan nachgewaschen, die Phasen getrennt und die wäßrige Phase erneut mit Pentan extrahiert. Die vereinigten Pentan-Phasen wurden 2 mal mit Wasser gewaschen, mit Natriumsulfat getrocknet und anschließend bei Normaldruck in eine gekühlte Vorlage destilliert.

Es wurden 103 g (56 % der Theorie) 1-Fluor-2,3-epithiopropan mit einem Siedepunkt von 98 bis 101° C (bei Normaldruck) erhalten.

Das $^{1}$H-NMR-Spektrum zeigte charakteristische Banden bei $\delta$ = 2,25, 2,55, 3,16, 4,25 und 4,54 ppm bei 200 MHz, gemessen in Deuterochloroform gegen Tetramethylsilan als internen Standard.

Das $^{19}$F-NMR-Spektrum zeigte eine charakteristische Bande bei $\delta$ = -127,3 ppm (Triplett) bei 75,4 MHz, gemessen in Deuterochloroform gegen Trifluoressigsäure als externen Standard.

Beispiel 2

50 g des gemäß Beispiel 1 hergestellten 1-Fluor-2,3-epthiopropans wurden destilliert und anschließend 24 Stunden bei 22° C stehen gelassen. Danach wurde die noch vorhandene Flüssigkeit abdekantiert und der verbleibende Feststoff im Vakuumtrockenschrank bei 50° C getrocknet. Dann wurde die Glastemperatur nach der DSC-Methode mit einer Heizrate von 20° pro Minute beginnend bei -100° C bestimmt. Es ergab sich ein Wert von -40° C.

**Patentansprüche**

**1.** 1-Fluor-2,3-epithiopropan der Formel

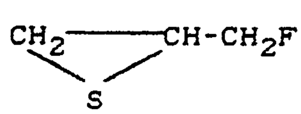

**2.** Verfahren zur Herstellung von 1-Fluor-2,3-epithiopropan, dadurch gekennzeichnet, daß man 1-Fluor-2,3-epoxypropan mit einem Schwefel übertragenden Agens in Gegenwart von Wasser und einem polaren organischen Lösungsmittel umsetzt.

**3.** Verfahren nach Anspruch 2, dadurch gekennzeichnet, daß man als Schwefel übertragendes Agens Kaliumthiocyanat, Ammoniumthiocyanat, Thiocyansäure, Thioharnstoff, Arylthioharnstoffe, Diarylthioharnstoffe, Thiocarbonsäuren und/oder Salze von Thiolsäuren einsetzt.

**4.** Verfahren nach Ansprüchen 2 und 3, dadurch gekennzeichnet, daß man 1,0 bis 2,5 Äquivalente Schwefel übertragendes Agens pro Mol 1-Fluor-2,3-epoxypropan einsetzt.

**5.** Verfahren nach Ansprüchen 2 bis 4, dadurch gekennzeichnet, daß man als polares organisches Lösungsmittel einen Alkohol mit 1 bis 6 C-Atomen einsetzt.

**6.** Verfahren nach Ansprüchen 2 bis 5, dadurch gekennzeichnet, daß die relativen Volumenmengen von Wasser zu polarem organischen Lösungsmittel 1:10 bis 10:1 betragen.

**7.** Verfahren nach Ansprüchen 2 bis 6, dadurch gekennzeichnet, daß man es bei 0 bis 50° C durchführt.

**8.** Elastomere, dadurch gekennzeichnet, daß sie erhältlich sind durch Stehenlassen von 1-Fluor-2,3-epithiopropan.

**9.** Elastomere nach Anspruch 8, dadurch gekennzeichnet, daß sie erhältlich sind durch Stehenlassen von 1-Fluor-2,3-epithiopropan für 10 bis 100 Stunden bei 10 bis 50° C.

**10.** Elastomere nach Ansprüchen 8 und 9, dadurch gekennzeichnet, daß sie erhältlich sind, indem man nach dem Stehenlassen noch vorhandene Flüssigkeit abtrennt und anschließend den verbleibenden Feststoff trocknet.